# EUROPÄISCHE PATENTSCHRIFT

(11) **EP 2 926 673 B1**
(45) Veröffentlichungstag und Bekanntmachung des Hinweises auf die Patenterteilung: **30.10.2019**
(21) Anmeldenummer: 14170676.2
(22) Anmeldetag: 30.05.2014
(51) Int. Cl.: A23L 2/44, A23L 2/56, A23L 27/00, A23L 27/12, A23L 3/349, A61K 8/60, A61K 8/31, A61K 8/34, A61Q 11/00, A61Q 13/00

(54) **Stoffgemische**
Compound mixtures
Mélanges de composés

(30) Priorität: 01.04.2014 US 201414973365
(43) Veröffentlichungstag der Anmeldung: 07.10.2015
(73) Patentinhaber: Symrise AG, 37603 Holzminden (DE)
(72) Erfinder: Krammer, Gerhard, 37603 Holzminden (DE); Siegel, Sven, 37671 Höxter (DE); Kennison, Deborah, Wayne, NJ 07470 (US)
(74) Vertreter: Fabry, Bernd

(56) Entgegenhaltungen:
- CN-A- 101 921 543
- US-A1- 2009 318 321
- US-A1- 2012 003 163
- US-A1- 2012 322 892
- US-A1- 2013 040 036
- US-A1- 2013 189 399
- US-A1- 2013 236 597
- US-A1- 2013 236 620
- US-A1- 2013 303 423
- MAYUKO NAKATA ET AL: "Volatile Components of Essential Oil from Cultivated Myrica gale var. tomentosa and Its Antioxidant and Antimicrobial Activities", JOURNAL OF OLEO SCIENCE, Bd. 62, Nr. 9, 1. Januar 2013 (2013-01-01) , Seiten 755-762, XP055146838, ISSN: 1345-8957, DOI: 10.5650/jos.62.755
- ANDREA BUETTNER ET AL: "Evaluation of Aroma Differences between Hand-Squeezed Juices from Valencia Late and Navel Oranges by Quantitation of Key Odorants and Flavor Reconstitution Experiments", JOURNAL OF AGRICULTURAL AND FOOD CHEMISTRY, Bd. 49, Nr. 5, 1. Mai 2001 (2001-05-01), Seiten 2387-2394, XP055146099, ISSN: 0021-8561, DOI: 10.1021/jf001363l

## Beschreibung

### GEBIET DER ERFINDUNG

Die Erfindung befindet sich auf dem Gebiet der Aromastoffe und betrifft neue Stoffgemische enthaltend Limonen, Propandiol-1,3 sowie Rebaudiosid A und Nahrungsmittel, die diese enthalten.

### STAND DER TECHNIK

Botanisch betrachtet stellen Zitruspflanzen eine Pflanzengattung aus der Familie der Rautengewächse *(Rutaceae)* dar, die ihre Heimat in den tropischen und subtropischen Gebieten Südostasiens haben. Aus der Sicht des Chemikers, zumal des Lebensmittelchemikers, stellen Zitrusfrüchte einzigartige Quellen zahlreicher monocyclischer Terpene dar, denen gemeinsam ist, dass sie über den einzigartigen Zitrusgeruch und -geschmack verfügen, der allgemein als Synonym für Frische und Reinheit gilt. Dass Zitrusaromen auch für den Feinschmecker ihre Bedeutung haben, zeigt, dass rund 20 % aller Interneteinträge zu diesem Thema einen Bezug zum Kochen aufweisen.

Insgesamt sind Zitrusaromen im Allgemeinen und monocyclische Terpene im Besonderen für die Aromastoffindustrie wichtige Verkaufsprodukte. Von erheblichem Nachteil ist dabei, dass die Stoffe allgemein sehr oxidationsanfällig sind. Von d-Limonen ist beispielsweise bekannt, dass es an der Luft zu Carvon, Carveol und Terpineol abgebaut wird. Dabei werden die fruchtigen, zitrusartigen und oranginen Noten durch die intensiv würzigen und krautigen Geschmacksnoten der Abbauprodukte überdeckt. Insbesondere tritt die kümmelartige Note des d-Carvons sehr schnell in den Vordergrund, was aus Sicht des Lebensmitteltechnikers, der ein Produkt mit Zitrusgeschmack komponieren möchte, außerordentlich unerwünscht ist.

Das Problem der mangelhaften Oxidationsstabilität von Terpenen ist literaturbekannt und somit hat es nicht an Versuchen gemangelt, den oxidativen Abbau der Stoffe zu verhindern oder wenigstens zu verlangsamen. Ansätze, bei denen man bekannte Antioxidatien, wie beispielsweise BHT, BHA oder Tocopherol zugegeben hat, haben sich als unbefriedigend erwiesen, zumal die Anwesenheit dieser Zusätze in Lebensmittel auch nicht erwünscht ist [vgl. Kimura et al., J. Agricult. Food Chem. 31, S.800-804 (1983**), *ibid.*** 47, 1661-1663 (1983**).].** Die internationale Patentanmeldung WO 1998 058656 A1 (Hauser) schlägt vor, die Zitruskomponenten durch Zugabe von Rosmarinsäure zu stabilisieren; doch auch diese Alternative erweist sich in der Praxis als nicht ausreichend.

Statt die Zitruskomponenten durch Zusätze zu stabilisieren, findet sich in der Literatur die Alternative, den Sauerstoff auszuschließen, indem man die Terpene verkapselt. So wird beispielsweise in der US 5,603,952 (Soper) vorgeschlagen, Terpene in Kapseln aus Fischgelatine einzuschließen. Allerdings wird damit das Einsatzgebiet der Produkte stark eingeschränkt, denn nicht in jeder Anwendung sind Kapseln gewünscht.

Gegenstand der US 2007 0116819 A1 sind Süßstoffzubereitungen, die im Wesentlichen die natürliche "High Potential Sweeteners" zusammen mit omega-ungesättigten Fettsäuren und gegebenenfalls Zusatzstoffen enthalten, die den Geschmack der Mischungen beeinflussen. Beispiel A1 offenbart ein Diätgetränk, welches als Süßstoff Rebaudiosid A enthält. Zur Steigerung der Süßwirkung wird Erythritol zugesetzt. In der Mischung ist auch Limonen enthalten, das aber nur als Dispergator fungiert.

Aus der WO 2005 048743 A1 sind Nahrungsmittel mit einer Fruchtkomponente bekannt, die verschiedene Frischekomponenten (z.B. Menthol) mit Kühlstoffen kombinieren. Es können auch Süßstoffe zugegen sein, wie beispielsweise Stevia. Der Prüfer führt diese Schrift als neuheitsschädlich gegen die Ansprüche 1, 4 und 13 an. Tabelle 6 offenbart eine Zubereitung mit Menthol und Stevia, wobei das Mengenverhältnis bei 0,25:99,75 beträgt.

Gegenstand der EP 2298084 A1 sind süßstoff-reduzierte Produkte, die natürliche Süßstoffe mit Phyllodulcin kombinieren. In Absatz 144 wird eine Zubereitung offenbart, die Rebaudiosid mit verschiedenen Kühlstoffen kombiniert, welche eine zyklische Monoterpenstruktur aufweisen. Das Gewichtsverhältnis Rebaudiosid zu Monoterpen liegt in der Formulierung A bei 13:87 und in Formulierung B bei 5:9.

Des Weiteren ist aus den beiden internationalen Patentanmeldungen WO 2013 134532 A1 und WO 2013 134607 A1 (KRAFT FOODS) bekannt, dass sich das Aroma von Nahrungsmitteln im Allgemeinen und Vollkornprodukten sowie Getränken im Besonderen durch Zugabe von 1,3-Propandiol verändern lässt. Die Schriften enthalten jedoch keinen Bezug auf die speziellen Probleme bei der Stabilisierung von monozyklischen Terpenen.

Die Aufgabe der vorliegenden Erfindung hat somit darin bestanden, den oxidativen Abbau von monozyklischen Verbindungen zu inhibieren oder wenigstens stark zurück zu drängen und auf diese Weise den Zitrusgeschmack in Nahrungsmittelzubereitungen über die Lagerzeit auf einem sensorisch gleichen Niveau zu halten oder sogar noch zu verstärken.

### BESCHREIBUNG DER ERFINDUNG

Gegenstand der Erfindung sind Stoffgemische, enthaltend
(a) Limonen,
(b) Propandiol-1,3 sowie
(c) Rebaudiosid A.

Überraschenderweise wurde gefunden, dass durch den Zusatz von Propandiol-1,3 der oxidative Abbau von Limonen stark vermindert wird. Die Restmengen an unerwünschten Abbauprodukten werden dann noch durch die Gegenwart des Diols maskiert. Im Ergebnis führt dies dazu, dass der Zusatz von Propandiol-1,3 zu Limonen sogar zu einer Verstärkung des Zitrusgeschmacks führt. Diese Effekte lassen sich weiter verstärken, wenn man den Stoffgemischen Rebaudiosid A zusetzt.

### TERPENE

Die meisten Terpene, speziell Mono- und Sesquiterpene leiten sich vom p-Menthan ab und weisen ein Cyclohexangerüst auf. Zu den wichtigsten Vertretern gehören neben dem Menthan, Phellandren, Terpinolen, Terpinen, Cymol und Limonen.

Im Sinne der Erfindung ist Limonen anwendungstechnisch von hoher Wichtigkeit und wird besonders leicht oxidativ abgebaut. Der Stoff kommt in zwei Enantiomeren vor, nämlich als (R)-(+)-Limonen (auch als D-(+) oder kurz (+)-Limonen bezeichnet) sowie als (S)-(-)-Limonen (auch als L-(-)-Limonen oder (-)-Limonen bezeichnet). Das Racemat der beiden Enantiomere wird auch Dipenten genannt.

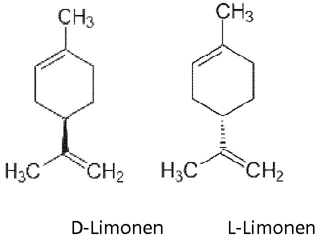

Im Sinne der vorliegenden Erfindung ist D-Limonen bevorzugt

### WIRKSTOFFE

Im Folgenden werden die Wirkstoffe näher erläutert.

**Rebaudioside** gehören zu den Steviosiden, den Hauptbestandteilen der Pflanze *Stevia rebaudiana,* die auch als Süßkraut oder Honigkraut bezeichnet wird.

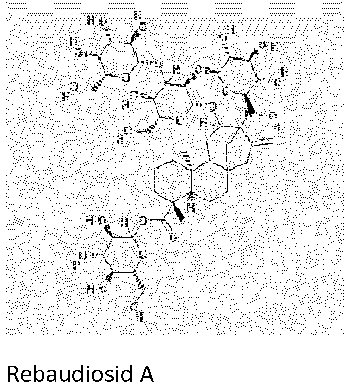

10 % der Trockenmasse der Blätter macht das Diterpenglykoid Steviosid aus, gefolgt von Rebaudiosid A (2 bis 4 Gew.-%) sowie zehn weiteren Steviolglycosiden, wie etwa dem Dulcosid. Rebaudioside und Stevia-Extrakt sind als Süßungsmitteln inzwischen in den meisten Staaten zugelassen; eine tägliche Aufnahmemenge von bis zu 4 mg Steviosid pro Kilogramm Körpergewicht wird als unbedenklich angesehen. Im Sinne der Erfindung können einzelne Rebaudioside oder die Extrakte der Stevia-Pflanze eingesetzt werden. Rebaudiosid A weist eine geringere Bitterkeit und die höchste Süßkraft aufw. Die erfindungsgemäßen Stoffgemische können die Komponenten (a) und (b) im Gewichtsverhältnis von etwa 1:99 bis etwa 99:1, vorzugsweise etwa 25:75 bis etwa 75:25 und insbesondere etwa 40:60 bis etwa 60:40 enthalten

ergänzend zu Rebaudiosid A können auch verschiedene andere Wirkstoffe eingesetzt werden, die ebenfalls die Zitrusnote verstärken, selbst aber kein Fruchtaroma aufweisen. Es sind dies:
- Monatin,
- Naringin,
- Chalkone und Hydrochalcone, insbesondere Dihydrochalcone,
- Mogroside sowie
- Extrakten der Pflanzen der Gattung *Rubus.*

**Monatin,** auch als Arruva bekannt, ist ein natürliches Süßungsmittel, das aus der Pflanze *Sclerochiton ilicifolius* isoliert werden kann.

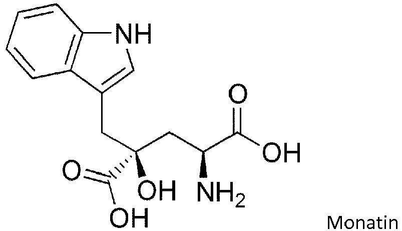

**Naringin** ist ein polyphenolisches Glykosid, das in der Grapefruit und dem Pomelo enthalten ist und diesen einen bitteren Geschmack verleiht. Der Stoff ist insbesondere wegen seiner lipidsenkenden Wirkung bekannt.

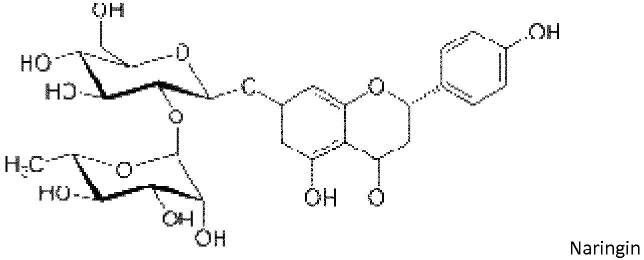

Auch die **Chalcone und Hydrochalcone** stellen Polyphenole dar, wobei insbesondere die Vertreter Naringin Dihydrochalcon und Neohesperidin Dihydrochalcon sowie das Phloretin hervorzuheben sind, die als künstliche Süßungsmittel bekannt sind:

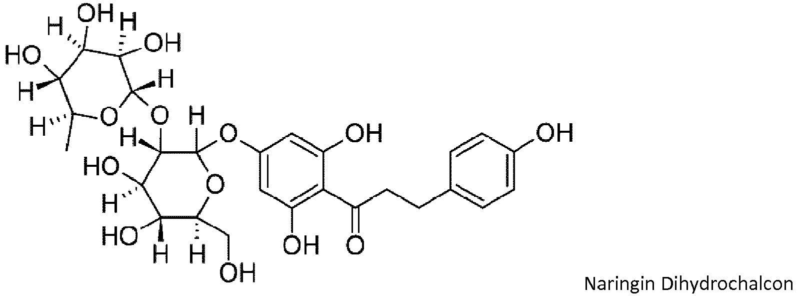

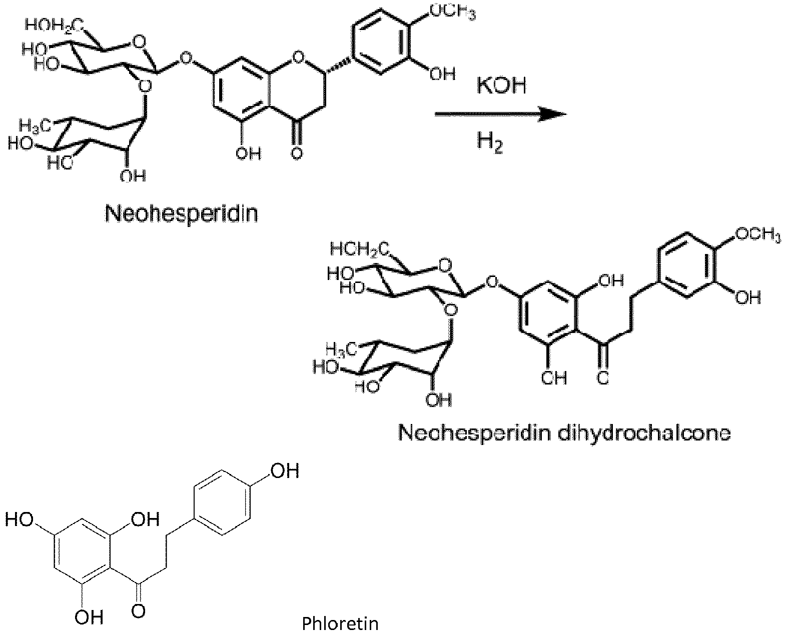

Unter der Bezeichnung **Mogroside** wird eine Gruppe von Glykosiden des Cucurbitans verstanden, die als Bestandteil des natürlichen Süßungsmittels Luo Han Guo (beispielsweise ex *Momordica grosvenorii)* bekannt sind. Hervorzuheben ist hier das Mogrosid-5, das 400mal süßer als Zucker ist.

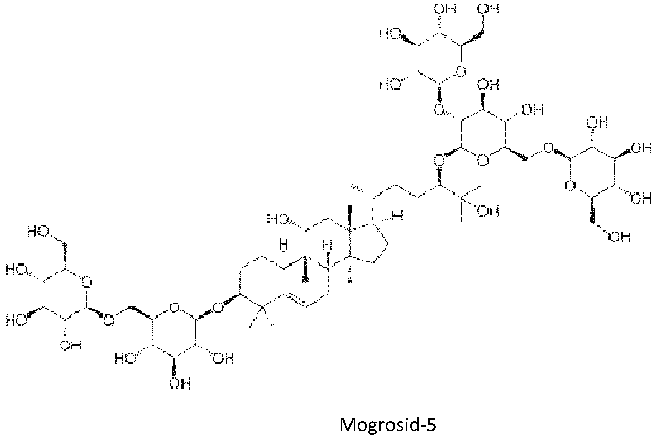

Schließlich kommen als Komponente (c) Extrakte der Pflanzen in Betracht, die ausgewählt sind aus der Gruppe, die gebildet wird von *Rubus allegheniensis, Rubus arcticu, Rubus strigosus, Rubus armeniacus, Rubus caesius, Rubus chamaemorus, Rubus corylifolius agg., Rubus fruticosus agg., Rubus geoides, Rubus glaucus, Rubus gunnianus, Rubus idaeus, Rubus illecebrosus, Rubus laciniatus, Rubus leucodermis, Rubus loganobaccus, Rubus loxensis, Rubus nepalensis, Rubus nessensis, Rubus nivalis, Rubus odoratus, Rubus pentalobus, Rubus phoenicolasius, Rubus saxatilis, Rubus setchuenensis, Rubus spectabilis* und *Rubus ulmifolius* sowie deren Gemischen. Hierbei handelt es sich im Wesentlichen um Extrakte von unterschiedlichen Brombeer- und Himbeerarten, die einen Gehalt an Rubosiden aufweisen. Bevorzugt sind Extrakte von *Rubus Suavissimus.*

In überraschender Weise wurde gefunden, dass der Zusatz von **Glycyrrhizinsäure** oder ein entsprechendes Salz oder einen Extrakt, der diesen Stoff enthält die Zitrusnote in den Stoffgemischen weiter verstärkt.

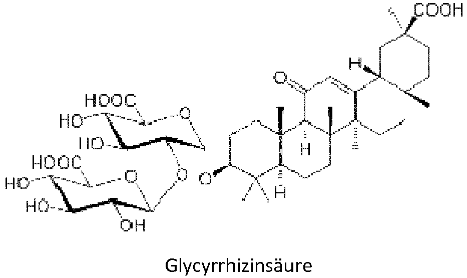

Auch dieser Befund ist für sich gesehen nicht vorhersehbar gewesen, denn die Glycyrrhizinsäure und die Glycyrrhinate weisen einen intensiven Lakritzgeschmack auf. Im Sinne der Erfindung ist es möglich, die Säure selbst, ihre Salze - beispielsweise Natrium-, Kalium- oder Ammoniumsalz - oder die Extrakte der Pflanze *Glycyrrhiza glabra* einzusetzen. Besonders bevorzugt ist das Monoammoniumglycyrrhizat.

### EXTRAKTIONSVERFAHREN

Sofern vorstehend auf Extrakte Bezug genommen worden ist, kann deren Herstellung in an sich bekannter Weise erfolgen, d.h. beispielsweise durch wässrigen, alkoholischen oder wässrig-alkoholischen Auszug der Pflanzen bzw. Pflanzenteile bzw. der Blätter oder Früchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden.

Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt.

Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 3 bis 15, insbesondere 6 bis 10 Gew.-%. Die vorliegende Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen, wobei ein intensiv rot gefärbter Feststoff zurückbleibt.

Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

### STOFFGEMISCHE

In einer ersten bevorzugten Ausführungsform können die Stoffgemische die Komponenten (a), (b) und (c) im Gewichtsverhältnis von 1:(0,1 bis 10):(0,1 bis 10) enthalten.

Die Stoffgemische können sich des Weiteren dadurch auszeichnen, dass sie einen Wassergehalt von weniger als etwa 2 und insbesondere von weniger als etwa 1 Gew.-% aufweisen. Vorzugsweise sind die Gemische völlig wasserfrei. Zu diesem Zweck können wässrige und/oder alkoholische Lösungen oder Dispersionen der Stoffgemische getrocknet werden, beispielsweise durch Sprühtrocknung, Wirbelschichttrocknung oder Lyophilisierung (Gefriertrocknung). Die trockenen Zubereitungen können anschließend entweder vermahlen oder granuliert werden.

### VERKAPSELUNG

Die Zubereitungen werden üblicherweise mit Hilfe von Festen Überzugsmaterialien verkapselt, wie beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nichtmodifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar.

Unter diesen Stoffen ist Gelatine besonders bevorzugt, da sie gut verfügbar ist und mit unterschiedlichen Schwellfaktoren bezogen werden kann. Ganz besonders bevorzugt, insbesondere für orale Anwendungen, sind nahtlose Gelatine- oder Alginatkapseln, deren Hülle sich im Mund oder beim Kauen sehr rasch auflöst oder aufbricht. Entsprechende Kapseln werden beispielsweise in den folgenden Schriften ausführlich EP 0389700 A1**,** US 4,251,195**,** US 6,214,376**,** WO 2003 055587 oder WO 2004 050069 A1**.**

Die Kapseln können alternativ auch Mikrokapseln darstellen. Unter den Begriffen "Mikrokapsel" oder "Nanokapsel" werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropy-Imethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind aus dem Stand der Technik hinlänglich bekannt **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a) aus Gelbildnern, kationischen Polymeren und Wirkstoffen eine Matrix zubereitet,
(b) gegebenenfalls die Matrix in einer Ölphase dispergiert,
(c) die dispergierte Matrix mit wässrigen Lösungen anionischer Polymere behandelt und gegebenenfalls dabei die Ölphase entfernt.

Die Schritte (a) und (c) sind dabei insofern austauschbar, als man anstelle der kationischen Polymeren in Schritt (a) anionische Polymere einsetzt und umgekehrt.

Man kann die Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.

### ZUBEREITUNGEN FÜR DIE ORALE AUFNAHME

Ein weiterer Gegenstand der Erfindung umfasst Zubereitungen für die orale Aufnahme, welche die erfindungsgemäßen Stoffgemische vorzugsweise in Mengen von 0,01 bis 1 Gew.-%, insbesondere etwa 0,05 bis etwa 0,5 Gew.-% und besonders bevorzugt von etwa 0,8 bis etwa 0,1 Gew.-% enthalten. Bei diesen Zubereitungen kann es sich zum einen um Nahrungsmittel, speziell Backwaren, Getränke, Kaugummis, Süßwaren und dergleichen oder um Mund- und Zahnpflegemittel handeln.

### Nahrungsmittel

Vorzugsweise handelt es sich bei den oralen Zubereitung um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

### Nahrungsmittelinhaltsstoffe

Die Nahrungsmittel können weitere Inhaltsstoffe aufweisen, wie z.B. Süßstoffe, Lebensmittelsäuren, Säureregulatoren, Verdickungsmittel und insbesondere Aromastoffe.

### Süßstoffe

Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

### In Betracht kommen auch

synthetische, d.h. in der Regel enzymatisch hergestellte Stärke oder Zuckerhydrolysate (Invertzucker, Fructosesirup);
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- Einzelsubstanzen wie z.B. Momordica grosvenori [Luo Han Guo] und die daraus gewonnenen Mogroside, Hydrangea dulcis oder Stevia ssp. (z.B. Stevia rebaudiana) Extrakte.

### Säureregulatoren

Die Nahrungsmittel können Carbonsäuren enthalten. Säuren im Sinne der Erfindung sind bevorzugt in Lebensmitteln zulässige Säuren, insbesondere die hier genannten:
- E 260: - Essigsäure
- E 270: - Milchsäure
- E 290: - Kohlendioxid
- E 296: - Apfelsäure
- E 297: - Fumarsäure
- E 330: - Citronensäure
- E 331: - Natriumcitrat
- E 332: - Kaliumcitrat
- E 333: - Calciumcitrat
- E 334: - Weinsäure
- E 335: - Natriumtartrat
- E 336: - Kaliumtartrat
- E 337: - Natrium-Kaliumtartrat
- E 338: - Phosphorsäure
- E 353: - Metaweinsäure
- E 354: - Calciumtartrat
- E 355: - Adipinsäure
- E 363: - Bernsteinsäure
- E 380: - Triammoniumcitrat
- E 513: - Schwefelsäure
- E 574: - Gluconsäure
- E 575: - Glucono-delta-Lacton

### Säureregulatoren

Säureregulatoren sind Lebensmittelzusatzstoffe, die den Säuregrad oder die Basizität und damit den gewünschten pH-Wert eines Lebensmittels konstant halten. Es handelt sich meist um organische Säuren und deren Salze, Carbonate, seltener auch um anorganische Säuren und deren Salze. Der Zusatz eines Säureregulators verstärkt teils die Stabilität und Festigkeit des Lebensmittels, bewirkt eine erwünschte Ausfällung und verbessert die Wirkung von Konservierungsmitteln. Im Gegensatz zu Säuerungsmitteln werden sie nicht zur Geschmacksveränderung von Lebensmitteln benutzt. Ihre Wirkung beruht auf der Bildung eines Puffersystems im Lebensmittel, bei dem sich auf Zugabe von sauren oder basischen Stoffen der pH-Wert nicht oder nur geringfügig ändert. Beispiele sind:
- E 170: - Calciumcarbonat
- E 260-263: - Essigsäure und Acetate
- E 270: - Milchsäure
- E 296: - Äpfelsäure
- E 297: - Fumarsäure
- E 325-327: - Lactate (Milchsäure)
- E 330-333: - Citronensäure und Citrate
- E 334-337: - Weinsäure und Tartrate
- E 339-341: - Orthophosphate
- E 350-352: - Malate (Äpfelsäure)
- E 450-452: - Di-, Tri- und Polyphosphate
- E 500-504: - Carbonate (Kohlensäure)
- E 507: - Salzsäure und ChlorideE 513-517Schwefelsäure und Sulfate
- E 524-528: - Hydroxide
- E 529-530: - Oxide
- E 355-357: - Adipinsäure und Adipate
- E 574-578: - Gluconsäure und Gluconate

### Verdickungsmittel

Verdickungsmittel sind Stoffe, die in erster Linie in der Lage sind, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Man spricht in diesem Fall davon, dass Moleküle miteinander 'kommunizieren', d.h. verschlaufen. Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder Ionenbeziehungen miteinander interagieren können. Extremfälle von Dickungsmitteln sind Schichtsilikate (Bentonite, Hectorite) oder hydratisierte SiO₂-Partikel, die als Teilchen dispergiert vorliegen und in ihrer festkörperartigen Struktur Wasser binden können bzw. aufgrund der beschriebenen Wechselwirkungen miteinander interagieren können. Beispiele sind:
- E 400: - Alginsäure
- E 401: - Natriumalginat
- E 402: - Kaliumalginat
- E 403: - Ammoniumalginat
- E 404: - Calciumalginat
- E 405: - Propylenglycolalginat
- E 406: - Agar Agar
- E 407: - Carrgeen, Furcelleran
- E 407: - Johannisbrotkernmehl
- E 412: - Guarkernmehl
- E 413: - Traganth
- E 414: - Gummi arabicum
- E 415: - Xanthan
- E 416: - Karaya (Indischer Traganth)
- E 417: - Tarakernmehl (Peruanisches Johannisbrotkernmehl)
- E 418: - Gellan
- E 440: - Pektin, Opekta
- E 440ii: - Amidiertes Pektin
- E 460: - Mikrokristalline Cellulose, Cellulosepulver
- E 461: - Methylcellulose
- E 462: - Ethylcellulose
- E 463: - Hydroxypropylcellulose
- E 465: - Methylethylcellulose
- E 466: - Carboxymethylcellulose, Natriumcarboxymethylcellulose

### Aromastoffe

Die Erfindung erlaubt insbesondere auch den Einsatz von Aromastoffen mit Ester-, Aldehyd- oder Lactonstruktur, die in Gegenwart von Titandioxid und unter Lichteinfluss besonders schnell abgebaut werden. Die Erfindung sorgt somit auch für eine verbesserte Stabilität, speziell Lagerstabilität der Aromastoffe.

Die erfindungsgemäßen oralen Zubereitungen können einen oder mehrere Aromastoffe enthalten. Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methylbuttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

### Kaugummis

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### Mund- und Zahnpflegemittel

Erfindungsgemäße oral konsumierbare süß schmeckende Produkte können auch der Mund- und Zahnreinigung und-pflege dienen. Beispiele hierfür sind Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stiviaextrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;

wundheilende und entzündungshemmende Stoffe wie z. B. Allantoin, Harnstoff, Azulen, Kamillenwirkstoffe und Acetylsalicylsäurederivate.

Eine bevorzugte Ausführung der oralen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### BEISPIELE

**Beispiele 4 und 5, Vergleichsbeispiele V1 und 1-3**

Verschiedene Stoffgemische auf Basis von Monoterpenen wurden zur Herstellung von Softdrinks verwendet und die Produkte 48 h bei 20°C gelagert. Anschließend wurden die geschmacklichen Eigenschaften von einem Panel bestehend aus 5 geschulten Testern auf einer Skala von 1 (nicht vorhanden) bis 10 (stark ausgeprägt) bewertet. Die Zusammensetzungen und Ergebnisse sind in der nachfolgenden **Tabelle 1** zusammengefasst. Es handelt sich um Mittelwerte von 5 Messreihen. Die Beispiele 4 und 5 sind erfindungsgemäß, Beispiel V1 und 1-3 dienen zum Vergleich. Das Beispiel C entspricht dem Standard, d.h. der geschmacklichen Beurteilung des Ausgangsproduktes unmittelbar nach seiner Herstellung.

**Tabelle 1**

| Geschmackliche Eigenschaften von Softdrinkformulierungen | | | | | | | |
|---|---|---|---|---|---|---|---|
| **Zusammensetzung** | **C** | **V1** | **1** | **2** | **3** | **4** | **5** |
| Sucrose | 10 | 10 | 10 | 10 | 10 | 10 | 10 |
| Zitronensäure | 0.2 | 0.2 | 0.2 | 0.2 | 0.2 | 0.2 | 0.2 |
| D-Limonen | 0.01 | 0.01 | 0.01 | 0.01 | 0.01 | 0.01 | 0.01 |
| Ribeaudiosid A | - | - | - | - | - | 0,01 | 0,01 |
| Propandiol-1,3 | - | - | 0,1 | 0,2 | 0,3 | 0,1 | 0,3 |
| Wasser | Ad 100 | | | | | | |

| ***Geschmackliche Beurteilung*** | | | | | | | |
|---|---|---|---|---|---|---|---|
| Zitrusnote | 6 | 4 | 8 | 9 | 9 | 9 | 9 |
| Krautnote | 0 | 4 | 3 | 3 | 3 | 2 | 1 |
| Kümmelnote | 0 | 4 | 3 | 3 | 2 | 2 | 1 |
| Bitterkeit | 4 | 5 | 3 | 3 | 2 | 1 | 1 |

Die Beispiele und Vergleichsbeispiele zeigen, dass die erfindungsgemäße Aufgabe vollumfänglich erfüllt wird: während die Ausgangsformulierung bei der Lagerung viel von ihrer Fruchtnote verliert und sich im Gegenzug ein krautiger Geschmack mit einer ausgeprägten Kümmelnote entwickelt, wird dies durch Zugabe geringer Mengen an Propandiol-1,3 nicht nur verhindert, die Zitrusnote wird sogar gesteigert.

### ZUSAMMENSETZUNGEN

**Tabelle 2a**

| Kaugummimassen | | | | | | | | |
|---|---|---|---|---|---|---|---|---|
| **Zusammensetzung** | **A** | **B** | **C** | **D** | **E** | **F** | **G** | **H** |
| Polyisobutylen (MW 20.000) | 30,0 | 30,0 | 30,0 | 40,0 | 20.0 | 20.0 | 25.0 | 30.0 |
| Glucose | 51,0 | 51,0 | 51,0 | 42,5 | - | - | - | - |
| Kornsirup | 10,0 | 10,0 | 10,0 | 8,0 | - | - | - | - |
| Sorbitol | - | - | - | - | 51,0 | 51,0 | 47,5 | 44,5 |
| Mannitol | - | - | - | - | 5,0 | 5,0 | 4,3 | 3,6 |
| Glycerin | 1,8 | 1,8 | 1,8 | 1,8 | 8,0 | 8,0 | 8,0 | 7,0 |
| Lycasin:Glycerin (1:1) | - | - | - | - | 8,2 | 8,2 | 8,0 | 7,0 |
| Lecithin | - | - | - | - | 0,2 | 0,2 | 0,2 | 0,2 |
| D-Limonen/Propandiol-1,3 (1:10) | 1,0 | 1,0 | 1,0 | 1,0 | 1,0 | 1,0 | 1,0 | 1,0 |
| Wasser | Ad 100 | | | | | | | |

**Tabelle 2b**

| Zusammensetzung Zahnpaste | | |
|---|---|---|
| **Komponente** | **Handelsprodukt** | **Menge [Gew.-%]** |
| Fällungskieselsäure | Sident® 12 DS | 18,0 |
| Verdickungskieselsäure | Aerosil® 200 | 0,8 |
| Sorbit | | 17,5 |
| Glycerin | | 17,5 |
| Carboxymethylcellulose | Relatin® 100 SR | 0,9 |
| Natriumlaurylsulfat | Texapon® K1296 | 2,0 |
| Natriumfluorid | | 0,22 |
| Saccharin-Natrium | | 0,2 |
| D-Limonen/Propandiol-1,3 (1:10) | | 1,0 |
| Wasser | | Ad 100 |

**Tabelle 2c**

| Zusammensetzung Mundwasser | | |
|---|---|---|
| **Komponente** | **Handelsprodukt** | **Menge [Gew.-%]** |
| | | |
| Ethanol (96%ig) | | 10,0 |
| Sorbitanmonolaurat+20E0 | Tween® 20 | 0,4 |
| D-Limonen/Propandiol-1,3 (1:10) | | 0,3 |
| Sorbit (70%ige wässrige Lösung) | | 8,0 |
| p-Hydroxybenzoesäuremethylester | | 0,2 |
| Wasser | | Ad 100 |

**Tabelle 2d**

| Zuckerfreies Bonbon | | | |
|---|---|---|---|
| **Komponente** | **X** | **XI** | **XII** |
| Isomalt | 94.0 | 94.0 | 94.0 |
| Xylitol | 2.40 | 2.40 | 2.40 |
| D-Limonen/Propandiol-1,3 (1:10) | 0.10 | 0,15 | 0.20 |
| Zitronensäure | 0.050 | 0.050 | 0.050 |
| Kirscharoma | 0.25 | 0.25 | 0.25 |
| Wasser | Ad 100 | | |

## Patentansprüche

1. Stoffgemische, enthaltend
(a) Limonen,
(b) Propandiol-1,3 sowie
(c) Rebaudiosid A.

2. Stoffgemische nach Anspruch 1, **dadurch gekennzeichnet, dass** sie als Komponente (a) D-Limonen enthalten.

3. Stoffgemische nach mindestens einem der Ansprüche 1 oder 2, **dadurch gekennzeichnet, dass** sie die Komponenten (a), (b) und (c) im Gewichtsverhältnis von 1:(0,1 bis 10):(0,1 bis 10) enthalten.

4. Stoffgemische nach mindestens einem der Ansprüche 1 bis 3, **dadurch gekennzeichnet, dass** sie einen Wassergehalt von weniger als 2 Gew.-% aufweisen.

5. Stoffgemische nach mindestens einem der Ansprüche 1 bis 4, **dadurch gekennzeichnet, dass** sie als Kapseln vorliegen.

6. Zubereitungen für die orale Aufnahme enthaltend die Stoffgemische des Anspruchs 1.

7. Zubereitungen nach Anspruch 6, **dadurch gekennzeichnet, dass** sie die Stoffgemische in Mengen von 0,01 bis etwa 1 Gew.-% enthalten.

8. Zubereitungen nach den Ansprüchen 6 und/oder 7, **dadurch gekennzeichnet, dass** sich um Backwaren, Getränke, Kaugummis, Süßwaren oder Mund- und Zahnpflegemittel handelt.

## Claims

1. Substance mixtures comprising
(a) limonene,
(b) propane-1,3-diol, and
(c) rebaudiosid A.

2. The substance mixtures according to claim 1, comprising D-limonene as component (a).

3. The substance mixtures according to at least one of claims 1 or 2, comprising components (a), (b) and (c) in a weight ratio of from 1:(0.1 to 10):(0.1 to 10).

4. The substance mixtures according to at least one of claims 1 to 3, having a water content of less than 2% by weight.

5. The substance mixtures according to at least one of claims 1 to 4, in the form of capsules.

6. Preparations for oral consumption comprising the substance mixtures according to claim 1.

7. Preparations according to claim 6, comprising the substance mixtures in an amount of from 0.01 to about 1% by weight.

8. Preparations according to claim 6 and/or claim 7, wherein the preparation is a bakery product, drink, chewing gum, confectionary product or oral or dental care composition.

## Revendications

1. Mélanges de substances contenant
(a) limonène;
(b) propanediol-1,3; et
(c) rebaudioside A.

2. Mélanges de substances selon la revendication 1, **caractérisés en ce qu'**ils contiennent du D- limonène comme composant (a).

3. Mélanges de substances selon au moins l'une des revendications 1 ou 2, **caractérisés en ce qu'**ils contiennent les composants (a), (b) et (c) dans un rapport pondéral 1 : (0,1 à 10) : (0,1 à 10).

4. Mélanges de substances selon au moins l'une des revendications 1 à 3, **caractérisés en ce qu'**ils présentent une teneur en eau inférieure à 2% en poids.

5. Mélanges de substances selon au moins l'une des revendications 1 à 4, **caractérisés en ce qu'**ils sont présents sous forme de capsules.

6. Les préparations pour administration orale contenant les mélanges de substances de la revendication 1.

7. Préparations selon la revendication 6, **caractérisées en ce qu'**elles contiennent les mélanges de substances en quantités de 0,01 à 1 % en poids.

8. Préparations selon les revendications 6 et/ou 7, **caractérisées en ce qu'**il s'agit de produits de boulangerie, de boissons, de gommes à mâcher, de confiseries ou de produits d'hygiène buccale et dentaire.
